# EUROPEAN PATENT SPECIFICATION

(11) **EP 1 684 730 B1**
(45) Date of publication and mention of the grant of the patent: **09.07.2008**
(21) Application number: 04793869.1
(22) Date of filing: 29.10.2004
(51) Int. Cl.: A61K 9/20, A61K 31/00

(54) **PHARMACEUTICAL COMPOSITION HAVING A CATIONIC EXCIPIENT**
PHARMAZEUTISCHE ZUSAMMENSETZUNG MIT EINEM KATIONISCHEN HILFSMITTEL
COMPOSITION PHARMACEUTIQUE CONTENANT UN EXCIPIENT CATIONIQUE

(30) Priority: 05.11.2003 SE 0302924
(43) Date of publication of application: 02.08.2006
(73) Proprietor: CAMURUS AB, S-213 75 Malmö (SE)
(72) Inventor: LJUSBERG-WAHREN, Helena, S-236 33 Höllviken (SE); LUNDBERG, Dan c/o Jan-Erik Lundberg, S-82193 Bollnäs (SE); HOLMBERG, Krister, S-413 10 Göteborg (SE)
(74) Representative: Presland, Torbjörn
(86) International application number: PCT/SE2004/001569
(87) International publication number: WO 2005/044237

(56) References cited:
- WO-A1-95/07692
- US-A- 5 492 937
- US-A- 6 007 826
- US-A- 6 056 938

## Description

### TECHNICAL FIELD

The present invention relates to the field of pharmaceutical compositions comprising a cationic excipient as a carrier ingredient. More specifically, the invention relates to a new group of cationic excipients for such compositions.

### BACKGROUND OF THE INVENTION

Positively charged molecules have over the years been evaluated as components of various types of drug delivery systems. Electrostatic interactions with the drug molecule, a component in the biological system or in some cases both are often essential for the mode of action of these types of excipients.

How fast a drug enters the systemic circulation after administration depends on several factors like the chemical characteristics of the substance (e.g. solubility, membrane permeability), the route of administration and the composition of the formulation. Selection of excipients for formulation of biological active materials like drugs must focus on therapeutic efficiency of the dosage form but the composition of the formulation can also affect other important factors like toxicity, drug load, storage stability and production costs.

There are several examples in the literature of cationic drug delivery systems containing lipids. It has for instance been suggested that by promoting nonbilayer structures in the cell membrane, lipids facilitate the intracellular delivery of macromolecules. Encapsulation in cationic liposomes has been shown to protect proteins and peptides against degradation by enzymes in biological fluids. Cationic lipid containing systems like emulsions and microemulsions have also been used to improve bioavailability after oral administration of sparingly water soluble drugs.

The development of novel drug formulations is often limited by the lack of safe and reliable excipients. A major problem with the use of surfactants in drug delivery systems is the potential toxicity of surface active agents. A balance between the efficacy in a carrier system for drugs and the toxic effects is always of major concern for substances involved in drug delivery. We have now found that a class of labile cationic substances, earlier demonstrated to have antimicrobial activity, have unique potential to be used as cationic excipients for drug delivery. The betaine ester, viz. betaine esterified with a lipophilic alcohol, is a cationic surfactant with outstanding drug delivery properties. Furthermore, the labile bond of the betaine ester results in a cationic hydrolysis product, betaine, which is a normal human metabolite. This means that the toxicity related to the cationic surfactant is transient.

The surface active betaine esters used in the present invention can together with drugs, and optionally other excipients, form aggregates like micelles, microemulsions, emulsions, dispersions and liquid crystalline phases in presence of water or biological fluids. Since betaine esters form complex with negatively charged polymers, like mucin, they are anticipated to retain the solubilized or dispersed drugs close to the absorption site without damaging the tissue. This is a feature that is especially interesting for transmucosal drug delivery. Transmucosal delivery at sites where enzymatic degradation can occur or which has a pH of 6.0 or higher should be of particular interest for drug delivery systems containing excipients with this type of labile esters. Drug delivery systems of negatively charged or sparingly water soluble drugs are also of special interest for this invention.

The use of a betaine ester as part of a carrier system for pharmaceutical applications is disclosed e.g. in US Patent No. 5,492,937 which describes a carrier composition which is a liquid at or below room temperature and forms a high viscosity layer or gel at body temperature, characterized in comprising a water-soluble, nonionic cellulose ether having a cloud point not higher than 40 °C, a charged surfactant and optional additives in water, wherein said optional additives are selected from the group consisting of flavouring agents, colorants, preservatives, isotonic agents and mixtures thereof, and in that the combined concentration of the water-soluble, nonionic cellulose ether and the surfactant is below 3% by weight, and wherein the remainder of the composition is water and said optional additives. The origin of the gel formation is a strong hydrophobic interaction between polymer and surfactant, which is cooperative in nature and thus resembles normal micelle formation. Surfactant clusters formed in this way may then act as cross-links between different polymer chains, giving rise to an extended three-dimensional gel structure. The surfactant should contain either a positively or a negatively charged headgroup. Examples of the former surfactants are alkyl ammonium compounds (e.g. hexadecyltrimethylammonium, tetradecylbetainate and hexadecylpyridinium salts, e.g. chloride and bromide). Thus, said carrier composition is aqueous and does not work the same way as the present invention.

US Patent No. 6,007,826 discloses a pharmaceutical or cosmetic composition comprising a pharmaceutically or cosmetically active effective amount of a hydrophobic active ingredient and a carrier, the carrier being an oil-in-water type emulsion which comprises colloid particles having an oily core surrounded by an interfacial film, said active ingredient being incorporated into said oily core, wherein said interfacial film comprises a combination of three different types of surface active compounds, a cationic lipid, a nonionic surfactant and an anionic surfactant or anionic lipid. Said cationic lipid is present in a concentration of 0.05-2% by weight and is selected from the group consisting of a C10-C24 primary alkylamine, a C10-C24 primary alkanolamine and a cholesterol ester (e.g. cholosteryl betainate). Cholesteryl betainate, a molecule with a large rigid steroid carbon ring structured with an esterified secondary alcohol has, however, properties quite different from those of the betainates used according to the present invention.

### SUMMARY OF THE INVENTION

The object of the invention is to provide a pharmaceutical composition comprising a pharmacologically active substance, generally a drug, and a carrier therefor comprising a new, specific type of cationic excipient which imparts good delivery or release characteristics to said composition.

Another object of the invention is to provide a composition the excipient of which is labile in the presence of water or aqueous body fluid so as to degrade into non-toxic products in the recipient body, generally a human being.

Still another object is to provide the use of said specific type of cationic excipient in a carrier for a pharmaceutical composition.

Other objects of the invention should be apparent to the reader of the more detailed disclosure of the invention presented herein.

The objects of the invention are achieved by a pharmaceutical composition as claimed in claim 1 and by the use as claimed in the independent use claims.

Preferable embodiments of the composition as well as the use referred to are as claimed in sub-ordinated claims or specifically disclosed in the specification below.

More specifically this means that the cationic excipient of the present invention is a labile ester of betaine and a lipophilic alcohol having at least one primary hydroxyl group.

It is preferred that the carrier or composition is substantially non-aqueous.

Said labile ester has been shown to work very well as a carrier for drug delivery and by said non-aqueous state of the carrier or composition the "lability" does not lead to any hydrolysis of the ester until in the animal (mammal), generally human, body. When degraded the ester will then result in the hydrolysis product betaine, which is a normal human metabolite. Thus, the toxicity generally related to the cationic surfactant can be said to be transient in connection with the present invention.

By "labile" in connection with the present invention is generally meant an ester which undergoes hydrolysis to more than 50% during 24 h at pH 7.4 in the presence of water or other aqueous liquid.

The term "substantially non-aqueous" generally means that such a condition does not cause any substantial hydrolysis of said labile ester, e.g. less than 10%, or less than 5%, in the composition referred to.

Another definition of "substantially non-aqueous" is that generally at most 5% by weight, preferably at most 2% by weight, most preferably at most 1% by weight, of water is present in the composition. A similar term having a similar meaning would be substantially water-free.

### BRIEF DESCRIPTION OF THE DRAWINGS

Fig. 1 shows results from Example 2 below.
Fig. 2 shows results from Example 4 below.

### DESCRIPTION OF PREFERABLE EMBODIMENTS

The labile betaine ester used according to the present invention is preferably an ester of betaine and an alcohol of the formula

R-CH₂-OH.

Thus, firstly a primary alcohol is used.

Secondly, R is a saturated or unsaturated aliphatic hydrocarbon residue, said unsaturation generally including double bonds only.

The carbon atom number of said symbol R is preferably 7 - 30, more preferably 7 - 22.

This in turn means that the cationic betaine esters referred to will have the formula:

R-CH₂-OCO-CH₂-N(CH₃)₂⁺X⁻

where X⁻ is a suitable counterion which is selected in accordance with known principles per se for surface-active cationic surfactants.

A suitable counterion may be chloride or bromide.

Like for all ionic surfactants the physico-chemical properties of a betaine ester surfactant are mainly governed by the length of the hydrophobic tail of the molecule, i.e. by the R residue in the above formula. The water solubility and the critical micelle concentration (CMC) of the betaine ester both decrease with increasing numbers of carbon atoms of R.

Versatile esters are esters wherein R has 9 - 13 carbon atoms.

As said, the alcohol referred to is a primary alcohol. It may, however, well also contain further hydroxyl groups, primary as well as secondary hydroxyl groups. That is, R is not necessarily an unsubstituted residue, but can be substituted, provided that the objects of the invention will not be lost. Expressed in another way, the substitution may even improve the characteristics of the cationic excipient, or composition, by the incorporation of substituents on R. Preferred substituents are primary and/or secondary hydroxyl groups, preferably one or two of each thereof.

Other substituents may be selected in accordance with known principles for cationic surfactants.

The hydrocarbon residue R need not either be a chain with carbon atoms only, i.e. a pure alkyl or alkenyl chain, but may well be interrupted by heteroatoms, such as O and/or N. According to a preferable embodiment of the invention at least one, preferably one or two, oxygen atom(s) is (are) present.

Preferably such oxygen atom(s) is (are) present in or as ester linkages.

More preferably said ester linkages are glyceride linkages, especially preferable examples being a 1-monoglyceride or a 2-monoglyceride.

Alternatively, the hydrocarbon residue R may comprise more than one tail, as exemplified by a diglyceride of the formula

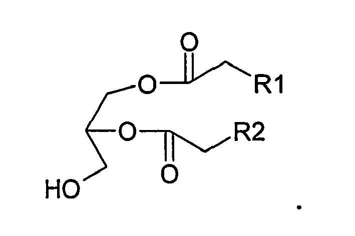

Alternatively, the hydrofobic moiety of the betaine ester may comprise more than one head group, as exemplified by a gemini surfactant 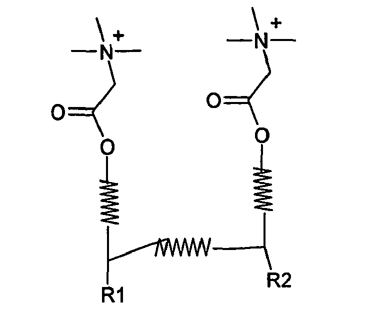 (a surfactant having two head groups and two hydrophobic tails joined by a short spacer).

The betaine esters of fatty alcohols are most conveniently prepared in a two-step reaction exemplified by the following chloride-based reaction. In the first step the fatty alcohol is reacted with chloroacetylchloride to form the chloroacetate. In a second step the chloroacetate is treated with trimethylamine to give the final product:

R-CH₂-OH + Cl-CH₂COCl → R-CH₂-OCO-CH₂-Cl

R-CH₂-OCO-CH₂-Cl + N(CH₃)₃ → R-CH₂-OCO-CH₂-N(CH₃)₃⁺ Cl⁻

Both steps usually proceed in good yields and the end product, the betaine ester, can be purified by recrystallization.

Other alcohols than fatty alcohols can be used as starting material for synthesis of surface-active betaine esters. Non-ionic surfactants and block copolymer surfactants, e.g. Pluronic®, which contains an alcohol group are also of interest for functionalisation with betaine. Alcohols from natural sources are of particular interest for betaine ester surfactants to be used in life science applications. Examples of such natural alcohols, besides fatty alcohols, are mono- and diglycerides. Betaine esters of such alcohols can be synthesized by the procedure described above but the first step, the formation of the chloroacetate intermediate, will demand higher temperature and/or longer reaction time when the alcohol is a secondary alcohol, as is the case for 1,3-diglyceride and cholesterol, or a primary alcohol with substituents on the α-carbon, as is the case for 1,2-diglyceride.

As mentioned above, betainates of straight-chain fatty alcohols can often be easily purified by recrystallization. When betaine esters are prepared from alcohols of natural origin, e.g. mono- or diglycerides, polymers containing hydroxyl groups or other alcohols with less well-defined structure, recrystallization of the product is often not possible. In these cases purification may be accomplished by for instance distillation or column chromatography of the intermediate or column chromatography of the final product. When using starting alcohols where the hydroxyl group is sterically hindered, as is often the case in the afore mentioned examples of glycerides and polymers, a quite low yield can be expected in the first reaction step. By using a large stoichiometric excess of trimethylamine, however, a very high conversion (close to 100%) may still be achievable in the second step. In such cases purification of the intermediate is an important part of the preparation procedure.

An alternative way of synthesizing surface-active betaine esters from a fatty alcohol is to first react betaine with chloroacetyl halogenide to form the acid halogenide, which in a subsequent step is reacted with the fatty alcohol. The reaction sequence is shown below for the chloride:

Cl-CO-CH₂-Cl + N(CH₃)₃⁺ → Cl-CO-CH₂-N (CH₃)₃⁺Cl⁻

R-CH₂-OH + Cl-CO-CH₂-N(CH₃)₃⁺Cl⁻ → R-CH₂-OCO-CH₂-N(CH₃)₃⁺Cl⁻

There exist yet other methods of preparing surface-active betaine esters. The present invention is not limited by the procedure used for the synthesis of the compound.
The betaine esters undergo alkaline hydrolysis much more readily than normal esters. On the other hand, they are more resistant to acid hydrolysis than normal esters. This pronounced pH-dependence of the hydrolysis is characteristic for this class of esters and is due to the structure of the molecules. The cationic charge in close proximity to the carbonyl carbon of the ester bond imparts electron deficiency to this carbon. Thus this carbon atom has a partial positive charge, which makes it a strong electrophile. Attack by a nucleophile at this carbon is therefore favoured compared to attack at a normal carbonyl carbon of an ester bond. An alternative or complementary way to account for the increased reactivity in alkaline hydrolysis of betaine ester bonds is to view the hydrolysis as a way to relief the strain of having a partial positive charge just two atom-atom bonds away from a permanent positive charge. The situation is shown below:

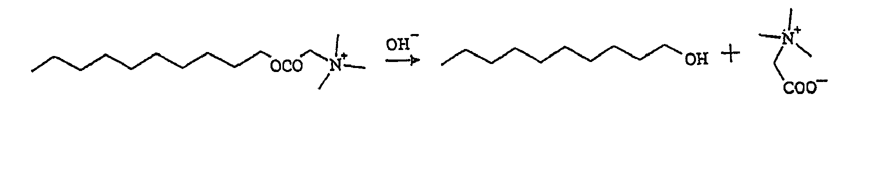

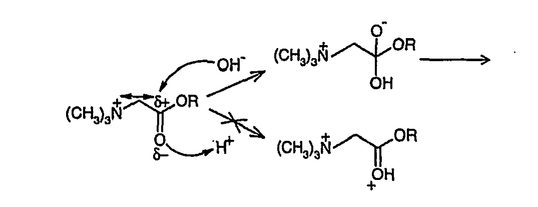

In these formulae it should be noted that R corresponds to R-CH₂- in previous formulae.

As is also shown in the reaction scheme, acid hydrolysis is "forbidden". It would yield a dicationic intermediate with the two positive charges close together and this is a very unfavourable arrangement. Taken together, the cationic charge in the betaine ester makes alkaline hydrolysis run very fast and acid hydrolysis extremely sluggish compared to hydrolysis of normal esters. The net effect is that betaine esters are most stable at very low pH, typically pH 2-3, and that the rate of hydrolysis is usually pH-dependent. Already at slightly alkaline conditions, there is substantial breakdown of the molecule.

Surface-active betaine esters are even more susceptible to alkaline hydrolysis than non-surface active esters. The reason for the increased reactivity of surface-active betaine esters is that the ester bond will hydrolyse more readily when the surfactants are in the form of aggregates, i.e. micelles. Like all other surfactants, surface-active betaine esters form micelles at a certain concentration, the CMC, and further increase of the surfactant concentration just leads to the formation of more micelles; the concentration of free surfactant molecules stays constant. In the vast majority of applications of cationic surfactants the concentration is far above the CMC, which means that almost all surfactants are in an aggregated form. The micelles of betaine ester surfactants are highly positively charged since all the quaternary ammonium groups are located at the surface of the aggregate. The positive charges must be compensated for by negatively charged ions, so-called counterions. All types of negative ions present in the solution, including hydroxyl ions, will accumulate at the micelle surface. This means that the local concentration of hydroxyl ions will be higher in the vicinity of the micelle than in the bulk, or, differently expressed, the local pH around the micelles will be higher than in the bulk. The higher pH will cause a more rapid ester hydrolysis of micellized surfactants than of free, non-aggregated surfactant molecules. This is referred to as micellar catalysis and is a well-known phenomenon in physical organic chemistry. As an example, the betaine ester of dodecanol at a concentration of 7.8 mM, which is 2.5 times the CMC, has a half-life of 90 minutes in a phosphate buffer of pD 7.5 at 37 °C (pD is the equivalent to pH in deuterated water, D₂O).

The extent to which micellar catalysis occurs is dependent on what other anions are present in the micellar solution. Large polarizable anions, such as bromide and iodide, will interact strongly with the micelle surface while smaller less polarizable anions, such as acetate, will have small affinity for the micelle. This means that hydroxyl ions will compete favourably, and be accumulated around the micelle, when the surfactant has a small anion, such as acetate, as counterion, but that they will not accumulate at the micelle surface if a large ion, such as iodide, is used as counterion. Use of different counterions for the betaine ester surfactant, and/or addition of extra salt to the surfactant solution, is therefore a way to tune the rate of hydrolysis.

In summary, the betaine ester surfactants break down rapidly on the alkaline side and are very stable on the acid side. The hydrolysis rate is unusually pH-dependent and is also governed by the type and concentration of anions in solution.

Cationic surfactants in general are known to interact strongly with surfaces and many of their technical applications, such as textile softener, additive to fluff and tissue, hair conditioner, corrosion inhibitor, etc., rely on strong adsorption to a surface. The reason why cationic surfactants adsorb particularly strongly is that the majority of surfaces are negatively charged, which means that a cationic surfactant can interact with the surface by both attractive electrostatic forces and by hydrophobic interactions. Also biological surfaces are usually negatively charged and the well documented antimicrobial action of cationic surfactants is due to a strong interaction with the lipid membranes of bacteria and other microorganisms. The strong interaction with biological lipid membranes is taken advantage of when cationic ampliphilic compounds are employed as bactericides and for gene transfection procedure mediated by cationic lipid vesicles. The strong interaction is also exploited in the use of cationic surfactants as carriers in intracellular delivery of bioactive agents, see US Patent No. 6,056,938. The interaction can also be a problem in that cationic surfactants usually have a higher dermatological toxicity than other surfactants.

Betaine ester surfactants have the same adsorption characteristics as normal cationic surfactants; thus, they adsorb strongly to negatively charged surfaces. The driving force for adsorption increases with the length of the hydrophobic tail of the surfactant, i.e. the R group of the surfactant of the formula above.

The driving force for adsorption of the betaine ester surfactants also depends on the ionic strength of the solution, the higher the electrolyte concentration, the stronger the adsorption. This is a common feature for all ionic surfactants.

The betaine esters will form aggregates such as monolayers, bilayers or hemimicelles at surfaces, including biological surfaces. It is very probable, that the ester will be subject to an increased rate of hydrolysis in such aggregates, in the same way as in aggregates in solution, i.e. micelles. Thus, "micellar catalysis" is likely to be an important element in the determination of the life-time of adsorbed surfactants. The betaine esters adsorbed at surfaces, as well as present in micelles in solution, are likely to be considerably more short-lived than free surfactant molecules in solution.

Various types of aggregates of the betaine esters, or small particles or droplets having a surface layer of the betaine esters, will also adsorb strongly to negatively charged surfaces. Since the cell walls are strongly negatively charged such aggregates or particles will bind strongly, and they will be retained at the cell surface. This is important for the use of these esters in drug delivery. The positively charged aggregates and small particles will also interact with negatively charged polyelectrolytes, such as cell surface mucins.

Preferable embodiments of the composition according to the invention are the following:

A water free composition which upon contact with water or other pharmaceutical relevant aqueous medium forms collodial particles and droplets.

A solid composition which upon contact with water or other pharmaceutical relevant aqueous medium forms collodial particles.

A composition which upon contact with water or other pharmaceutical relevant aqueous medium forms collodial particles, the pharmacologically active substance being a negatively charged substance or a substance having low water solubility (more than 250 ml water is needed to dissolve the highest dose strength).

A composition which upon contact with water or other pharmaceutical relevant aqueous medium forms micelles, a microemulsion, an emulsion or a dispersion of a liquid crystalline phase.

A composition which upon contact with water or other pharmaceutical relevant aqueous medium forms a dispersion of a cubic, lamellar or hexagonal liquid crystalline phase.

In this context collodial particles generally means a particle size less than 10 µm.

Furthermore, the composition referred to here and otherwise in the specification means a composition containing the pharmacologically (biologically) active substance, the betaine ester and optionally other conventional pharmaceutical excipients, such as non-ionic surface active compunds, and/or solvents.

The compositions of the present invention can be used for improved delivery of hydrophilic or hydrophobic pharmacologically active substances. The invention is not limited to the use of any specific substances but preferably do the biologically active substances have low water solubility or are negatively charged substances. Examples of biologically active substances include, but are not limited to, nucleic acids such as DNA, cDNA, RNA (full length mRNA, ribozymes, antisense RNA), oligodeoxynucleotides (phosphodiesters, phosphothioates, phosphoramidites, and all other chemical modifications), oligonucleotide (phosphodiesters, etc.) or linear and closed circular plasmid DNA; negatively charged proteins and carbohydrates including polysaccharides. Suitable drugs include antivirals (acyclovir, IUdR, ganciclovir, vidarabine, AZT), steroidal and non-steroidal antiinflammatory drugs (dexamethasone, loteprednol, prednisolone derivatives, diclofenac, indomethacin, piroxicam etc.), antibiotics (e.g., ampicillin and erythromycin) antifungals (e.g., miconazole), vitamins, hormones, retinoic acid, local anesthetics, calcium channel blockers (e.g., Verapamil), prostaglandins and prostacyclins, antineoplastic and antimetabolitic drugs, miotics, cholinergics, adrenergic antagonists, anticonvulsants (e.g., phenytoin), antianxiety agents, major tranquilizers, antidepressants, anabolic steroids, estrogens, progesterones, and glycosaminoglycans (heparin, heparan, chondroitin sulfate, and low molecular weight derivatives thereof).

"Ionic interaction" or "electrostatic interaction" refers to intermolecular interaction among two or more molecules, each of which is positively or negatively charged. Thus, for example, can positively charged lipids interact with negatively charged molecules like DNA. Gene transfer represents an important advance in the treatment of both genetic and acquired diseases. Cationic lipid-mediated gene transfer have advantages over viral gene transfer due to their non-immunogenic properties.

Many of the drugs listed above have low bioavailably when administered orally due to low water solubility, slowly transport through mucous, low permeability through the epithelial cells, instability in biological fluids or a combination of these factors. Low bioavailability of such drugs severely limits their applicability, usage and effectiveness.

Typical pharmaceutical applications of the invention are in oral administration or transmucosal delivery of sparingly water soluble drugs. For oral administration a composition containing the drug and the betaine ester is encapsulated in a sealed soft or hard gelatin capsule. The capsule is typically of a kind which is dissolved in a particular region of the GI tract where it releases its content. Examples of such capsules are entero-coated soft or hard gelatin capsules. Enteric coating, as known per se, is a coating consisting of a substance or a combination of substances that resists dissolution in gastric fluid but disintegrates in the intestine. The formation of well-defined colloid drug containing particles and droplets (e.g. liposomes, microemulsions, emulsions, Cubosome® or Hexosome® particles) when the capsule is disintegrated brings about predictable release of the drug which may offer an improvement in both the rate and extent of absorption. Esters of long chained fatty acids in lipid drug delivery dispersions will after digestion and absorption be transported via the lymphatic system in so called chylomicrons. The chylomicrons are in turn carried away from the small intestine through the thoracic duct, thus bypassing the liver. Such an absorption route thus significantly reduces the first pass effect of drug absorbed together with the lipids.

Dosage forms of the compositions of pharmacologically active substances, betaine esters and any other excipients can be fluid, semisolid or solid. Betaine esters and biologically active substances my be combined with other excipients so that they are fluid at elevated temperature which allows for filling capsules followed by formation of a solid solution, a solid dispersion or a semisolid formulation when the capsules are stored at room temperature. The term semisolid should be interpreted in the common way in this technical field, i.e. generally a formulation that does not flow under its own weight. Normally this also means that it is semisolid at room or ambient temperature and can be liquefied at higher temperatures.

Inclusion of surfactants in lipid-based liquid crystalline drug delivery particles or precursor systems of such particles can improve loading of water soluble drugs. The enhanced loading of the negatively charged water-soluble drug ketoprofen by the inclusion of cationic surfactants into Cubosome® particles have been demonstrated in the literature. Development of lipid-based particles like Cubosome®, Hexosome® and liposomes containing the betaine esters and water soluble drugs is an application of the invention. Another interesting application of the invention is formation of positively charged aggregates of a dispersed lamellar liquid crystalline phase containing the betaine ester and drug that with time undergoes phase transition due to hydrolysis of the betain ester, thereby altering the drug delivery properties of the particles.

In one embodiment of the invention the betaine ester is combined with other lipid excipients like PC in order to lower the toxicity of the drug delivery vehicle. The reduction in toxicity may be evaluated by haemolysis experiments.

DNA transfection efficiency of aggregates containing cationic lipids can be modified by co-formulating with neutral "helper lipids" like dioleoylphosphatidyl-ethanolamine (DOPE), cholesterol or poly(ethylene glycol)-phospholipid conjugate. Of special interest is co-formulation of the betaine esters with polar lipids that promote formation of non-lamellar structures, e.g. phosphatidylethanolamine (PE).

Cryoprotectants and polymers are examples of components that may optionally be used in the drug delivery systems based on betaine esters. A cryoprotectant or anticoalescent compound may be added to a formulation of betaine ester and drug prior to dehydration/evaporation to inhibit flocculation and coalescence upon rehydration. The cryoprotectant may be of any type known in the art, including sugars and polysaccharides such as sucrose or trehalose, and nonnatural polymers such as polyvinylpyrrolidone. Cryoprotectants are usually present at less than 25%, commonly 10%, more commonly 5%, 4% (w/v) or less in the emulsion before lyophilization. Natural polymers, synthetically modified natural polymers, such as (hydroxypropyl) methylcellulose or synthetic polymers, such as polyvinylalcohol may also be included in betaine ester formulations in order to modify the release of the drug carrying aggregate/particle

The betaine ester-containing composition according to the invention may be prepared by use of water or other solvents followed by evaporation, wherein the evaporation is accomplished by spray drying, freeze drying, air drying, vacuum drying, fluidized bed drying, coprecipitation, or super-critical fluid evaporation.

A further aspect of the invention provides a dehydrated colloidal suspension. Dehydrated suspensions may be stored for prolonged periods with minimal degradation, and can be reconstituted with water shortly before use. The residual water content in an dehydrated emulsion is usually less than 5% (w/w), commonly less than 2%, and often less than 1%.

Dehydration may be performed by standard methods, such as drying under reduced pressure; when the suspension is frozen prior to dehydration, the low pressure evaporation is known as lyophilization. Freezing may be performed in a dry ice-acetone or ethyl alcohol bath. The pressure reduction may be achieved with a mechanical vacuum pump, usually fitted with a liquid nitrogen cold trap to protect the pump from contamination. Pressures in the low millitorr range, e.g., 10-50 millitorr, are routinely achievable, but higher or lower pressures are sufficient.

Especially preferable embodiments of the invention are compositions in the form of freeze-dried powder, spry-dried powder and a pumpable mass that can be filled into a capsule.

The pharmacologically effective amount of the active substance is of course chosen, by the person skilled in the art, along known principles, while taking into consideration which specific compound is selected, the specific use therof and so on. Similarly the concentrations of excipients, solvents, etc. are also selected in accordance with prior art so as to achieve the desired solid, semisolid or fluid state. Finally, the percentage of the betain ester is also easily determined by the skilled artisan while considering known principles concerning cationic excipients and the specific purposes to be obtained.

### EXAMPLES

### Example 1:

### Synthesis of dodecyl betainate

Dodecyl betainate was prepared from dodecanol, choloroacetyl chloride and trimethylamine using the two-step synthetic procedure described below:

In the first step chloroacetyl chloride (14.2 g, 126 mmol) in dichloromethane (25 ml) was drop-wise added to a stirred solution of 1-dodecanol (22.73 g, 122 mmol) in dichloromethane (100 ml). The reaction mixture was stirred for 6 h at room temperature and then gently refluxed for 0.5 h. After being washed with a 5% solution of sodium hydrogen carbonate (3x25 ml), to remove excess chloroacetyl chloride, the organic phase was dried over magnesium sulphate, filtered and rotary evaporated.

In the second step trimethylamine (14.0 g, 237 mmol) was slowly bubbled through an ice-cooled, stirred solution of dodecyl chloroacetate (15.24 g, 58 mmol) in dry acetone (600 ml), whereupon the solution was allowed to attain room temperature. After 76 h the product, a white fluffy precipitate, was collected on a glass filter and washed with diethyl ether (3x20 ml). The product weighed 16.2 g, corresponding to an overall yield of 83%. ¹H-NMR (CDCl₃): δ 0.88 (t, 3H), 1.22-1.39 (m, 18H), 1.66 (m, 2H), 3.66 (s, 9H), 4.18 (t, 2H), 5.19 (s, 2H)

### Example 2:

### Concentration dependence on the hydrolysis of surface active betaine esters

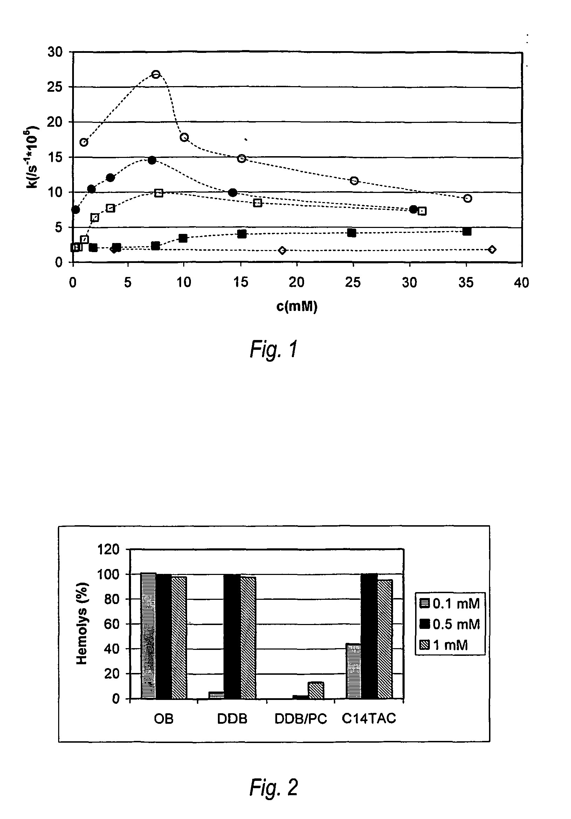

Figure 1 shows the concentration dependency of the hydrolysis rate for surface active betaine esters, exemplified by the initial pseudo first-order rate constants versus concentration for a number of betaine esters ((o) Oleyl betainate, (•) tetradecyl betainate, (□) dodecyl betainate, (■) decyl betainate, (◇) ethyl betainate) in a phosphate buffer of pD=7.5 at 37 °C (pD is the equivalent to pH in deuterated water, D₂O). Ethyl betainate is included as a non surface active reference. The increase in hydrolysis rate with increasing concentration is caused by an increasing contribution from micellar catalysis, and the following decrease can be explained by the increased competition between the hydroxyl ions and the surfactant counterions at the micellar surface.

Figure 1 also shows the dramatic increase in hydrolysis rate due to the presence of micellar catalysis for the surface active betaine esters. For instance, dodecyl betainate at a concentration of 7.8 mM, which is 2.5 times the CMC, has a half-life of 90 minutes, compared to a half-life of 9 h for ethyl betainate.

### Example 3:

### Ussing chamber experiments to show low toxicity of betainate systems

A modified Ussing diffusion chamber with an exposed tissue area 1.78 cm² was used in the experiments. 15 to 20 cm of the small intestine, distal to the Ligament of Treitz was removed from rats (male Sprague-Dawley, 400-500 g) and used in the tests. Three rats and three segments per rat were included in each experimental group. The passage of different marker molecules, ¹⁴C-mannitol and FITC-dextran (4400 FD4), from the mucosal to the serosal chamber were expressed as apparent permeability coefficient (Papp). The apparent permeability coefficient (Papp) to mannitol and FITC-dextran 4.400 across the intestinal mucosa was calculated from the equation: Papp (cm/s) x 10⁻⁶) = dc/dt * (V/(A*C0)), where dc/dt is the change of the serosal concentration over time (mol/L/sec), V is the volume in the reservoir of the serosal side (cm³), C0 is the initial concentration of the marker in the mucosal reservoir (mol/L), and A is the exposed intestinal area in the chamber (cm²). The experiments were done on marker molecules dissolved in a dodecyl betainate (0.5 % w/w) containing solution and in a control solution(Krebs buffer).

The Papp values for 14C-mannitol are scattered between 3 and 4 x 10⁻⁶ cm/s. There are no obvious differences between the different treatments.

The Papp values for FD4 are scattered between 0.4 and 0.6 x 10⁻⁶ cm/s (App. 2). There are no obvious differences between the different treatments.

The difference in electric potential over the segments was measured before and after the experiment. With respect to permeability for marker molecules, the intestinal segments were not affected by co-formulation with dodecyl betainate. Furthermore there was no increased damage to segments in response to treatment with betaine ester containing solution as compared to control, assessed by determination of potential difference over the segments before and after treatment.

### Example 4:

### Hemolysis experiments on betaine ester surfactants

Erythrocytes from rats were washed 3 times and suspended in saline solution (0.9%) to a volume fraction of 0.025. The hemolys experiments were performed by mixing the erythrocyte suspension with surfactant solutions in a 1:1 ratio, thereby giving a finale erythrocyte volume fraction of 0.0125 and a surfactant concentration as stated in the figure. The samples were then incubated at 37 °C for 1 hour and centrifuged at 2000 g for 10 minutes. Aliquots of the supernatant was mixed with an equal volume of 15 mM C14TAC before the hemoglobin content was measured spectrophotometrically on a Labsystems iEMS Reader MF at 540 nm.

For the samples containing phospholipids, a liquid concentrate of dodecyl betainate, PC and ethanol in a weight ratio of 16:64:20 were prepared. The concentrate was dispersed in saline solution by gentle stirring at room temperature just before the experiment. The resulting aqueous dispersion was tested in the same way as stated above for the surfactant solution.

Figure 2 shows the relative haemolytic effect of different cationic surfactant solultions and a cationic ionic phospolipid dispersion. The concentrations given in the figure represent the amounts of cationic surfactant in the tested solutions. OB = oleyl betainate, DDB = dodecyl betainate, DDB/PC = dodecyl betainate coformulated with phosphatidyl choline in a weight ratio of 20 to 80 (concentration of DDB given), C14TAC = tetradecyltrimethylammonium chloride. The phosphatidyl choline used is Epikuron 200 from Degussa BioActives, a pharmaceutical grade product of soybean origin. All data are normalized to the absorbance obtained from hemolysis by the 0.5 mM tetradecyltrimethylammonium chloride sample.

This example shows that at low concentrations the toxicity to red blood cells of dodecyl betainate is lower than that of the stable surfactant C14TAC, which has a comparable critical micelle concentration 2.5 mM (CMC for dodecylbetainate is 3.1 mM) and that the toxicity of can be further lowered by co-formulating the betaine ester with phosphatidyl choline.

### Example 5:

### Aqueous particle dispersion of a sparingly water soluble substance stabilized with betaine esters.

The ability of dodecyl betainate to stabilize aqueous dispersions of hydrophobic particles was investigated in samples containing 1 % cholesterol (of lanolin origin, Fluka) in pure water and in dodecyl betainate solutions of varying concentration. The samples were prepared by weighing the components into screw-capped glass vials that were first shaken, then kept in an ultrasonic bath for 5 minutes and finally shaken again before left for inspection. The table below presents the visual appearance of samples with different surfactant concentrations at different times.

| Dodecyl betainate conc. | Visual appearance after 5 min. | Visual appearance after 60 min. |
|---|---|---|
| 0 | Coarse dispersion of flake-like particles | Almost clear water and precipitate |
| 0.1% w/w (3 mM) | Turbid dispersion | Very slightly turbid dispersion and precipitate |
| 0.3% w/w (9 mM) | Highly turbid dispersion | Highly turbid dispersion and some precipitate |

The sample with pure water was a coarse dispersion that precipitated quickly, while samples containing more than approximately 0.1% of the surfactant were fine dispersions with prolonged stability. The table shows that particles of water insoluble particles like steroids can easily be stabilized against particle aggregation and sedimentation by betainate esters in aqueous systems.

### Example 6:

### Transformation from a lamellar liquid crystalline phase to a cubic phase with time in a mixture of dodecyl betainate, glycerol monooleate and a physiologically relevant medium

A solution A was made from 81% w/w glycerol monooleate (Danisco Brabrant, Danmark), 9% w/w dodecyl betainate and 10% w/w ethanol. Two samples were prepared by mixing solution A with simulated intestinal fluid (SIF) (pH = 7.4) and with water, respectively. The compositions of the samples are reported in the table below. The phase behaviour of the samples as a function of time was studied by visual inspection between crossed polarizers and reported in the table below. A sample of glycerol monooleate and water was used as a reference. Glycerol monooleate is known to form a cubic, nonbirefingent liquid crystalline phase in excess water at room temperature.

| Composition | Time 0 h | Time 0.5 h | Time 14 h |
|---|---|---|---|
| 0.25 g A/1.8 g water | Dispersion | Homogenous birefingent | Homogenous, birefingent |
| 0.27 g A/1.8 g SIF | Dispersion | Homogenous, birefingent | Two non birefingent phases |
| Glycerol monooleate 0.3 g /water 1.8 . | - | - | Two non birefingent phases |

This experiment shows that addition of dodecyl betainate to glycerol monooleate induces a phase shift from a cubic phase to a birefingent phase, probably a lamellar phase and that this lamellar phase at physiological relevant conditions with time transforms into two phases, probably cubic liquid crystalline phase and an aqueous solution.

## Claims

1. A pharmaceutical composition comprising a pharmaceutically effective amount of a pharmacologically active substance and a pharmaceutically acceptable carrier comprising a cationic excipient, wherein said cationic excipient is a labile ester of betaine and a lipophilic alcohol having at least one primary hydroxyl group.

2. A composition according to claim 1, wherein said carrier is substantially non-aqueous.

3. A composition according to claim 1 or 2, wherein said composition is substantially non-aqueous.

4. A composition according to any one of the preceding claims, wherein said lipophilic alcohol is an alcohol of the formula R-CH₂-OH where R is a saturated or unsaturated aliphatic hydrocarbon residue having 7 - 30, preferably 7 - 22, carbon atoms.

5. A composition according to claim 4, wherein said hydrocarbon residue has at least one primary and/or at least one secondary hydroxyl groups as substituent(s).

6. A composition according to claim 5, wherein said primary and/or secondary hydroxyl groups are each at most two.

7. A composition according to any one of claims 4 - 6, wherein said hydrocarbon residue is interrupted by at least one, preferably one or two, oxygen atoms.

8. A composition according to claim 7, wherein said oxygen atom(s) is (are) present in an ester linkage.

9. A composition according to claim 8, wherein said ester linkage is present in a glyceride.

10. A composition according to claim 9, wherein said glyceride is a 1-monoglyceride or a 2-monoglyceride.

11. A composition according to any one of claims 4-10, wherein said unsaturated hydrocarbon residue is a residue containing one or two double bonds.

12. A composition according to any one of the preceding claims, wherein said pharmacologically active substance has low water solubility, such as cyklosporine or an analogue thereof.

13. A composition according to any one of claims 1 - 12, wherein said pharmacologically active substance is negatively charged, such as a negatively charged protein, a negatively charged carbohydrate, preferably low molecular weight derivatives of heparin, or DNA.

14. A composition according to any one of preceding claims, which has a water content of at most 5% by weight, preferably at most 2% by weight and most preferably at most 1% by weight.

15. A composition according to any one of the preceding claims, which is solid, semi-solid or liquid, preferably solid or semi-solid.

16. A composition according to claim 15, which is in the form of a powder or a waxy powder, preferably a freeze-dried or spray-dried powder.

17. A composition according to claim 15, which is a pumpable mass that can be filled into a capsule.

18. A composition according to any one of the preceding claims, which upon contact with water or other aqueous medium forms collodial particles, preferably a solid, especially water free, composition.

19. A composition according to claim 18, wherein said pharmacologically active substance is a negatively charged substance or a substance having a low water solubility.

20. A composition according to any one of claims 1 - 17, which upon contact with water or other aqueous medium forms micelles, a microemulsion, an emulsion or a dispersion of a liquid crystalline phase.

21. A composition according to one of claims 1 - 17, which upon contact with water or other aqueous medium forms a dispersion of a cubic, lamellar or hexagonal liquid crystalline phase.

22. A composition according to any one of the preceding claims, wherein said carrier also comprises an excipient selected from polymers, lipids, carbohydrates, non-ionic surface active compounds and mixtures thereof.

23. A composition according to claim 22, wherein said carbohydrate is a low molecular carbohydrate, preferably selected from lactose, sucrose, maltose and trehalose.

24. A composition according to claim 22 or 23, wherein said lipid is selected from phospholipids, cholesterol and glycerides from medium- or long-chained fatty acids.

25. Use of a labile ester of betaine and a lipophilic alcohol having at least a primary hydroxyl group as a cationic excipient in a carrier for a pharmaceutical composition comprising a pharmacologically active substance.

26. Use according to claim 25, wherein said carrier is substantially non-aqueous.

27. Use according to claim 25 or 26, wherein said composition is substantially non-aqueous.

28. Use according to any one of claims 25 - 27, wherein said alcohol is as defined in any one of claims 4 - 11.

29. Use according to any one of claims 25 - 28, wherein said composition is as defined in any one of claims 12 - 24.

## Patentansprüche

1. Pharmazeutische Zusammensetzung umfassend eine pharmazeutisch wirksame Menge eines pharmakologischen Wirkstoffs und einen pharmazeutisch annehmbaren Träger umfassend einen kationischen Exzipienten, wobei der kationische Exzipient ein labiler Ester von Betain und einem lipophilen Alkohol mit wenigstens einer primären Hydroxylgruppe ist.

2. Zusammensetzung nach Anspruch 1, wobei der Träger im Wesentlichen nicht-wässrig ist.

3. Zusammensetzung nach Anspruch 1 oder 2, wobei die Zusammensetzung im Wesentlichen nicht-wässrig ist.

4. Zusammensetzung nach einem der vorangehenden Ansprüche, wobei der lipophile Alkohol ein Alkohol der Formel R-CH₂-OH ist, wobei R ein gesättigter oder ungesättigter aliphatischer Kohlenwasserstoffrest mit 7 - 30, vorzugsweise 7 - 22 Kohlenstoffatomen ist.

5. Zusammensetzung nach Anspruch 4, wobei der Kohlenwasserstoffrest wenigstens eine primäre und/oder wenigstens eine sekundäre Hydroxylgruppe als Substituent(en) aufweist.

6. Zusammensetzung nach Anspruch 5, wobei die primären und/oder sekundären Hydroxylgruppen jeweils höchstens zwei sind.

7. Zusammensetzung nach einem der Ansprüche 4 - 6, wobei der Kohlenwasserstoffrest durch wenigstens ein, vorzugsweise ein oder zwei Sauerstoffatome unterbrochen ist.

8. Zusammensetzung nach Anspruch 7, wobei das Sauerstoffatom (die Sauerstoffatome) in einer Esterbindung vorhanden ist (sind).

9. Zusammensetzung nach Anspruch 8, wobei die Esterbindung in einem Glycerid vorhanden ist.

10. Zusammensetzung nach Anspruch 9, wobei das Glycerid ein 1-Monoglycerid oder ein 2-Monoglycerid ist.

11. Zusammensetzung nach einem der Ansprüche 4 -10, wobei der ungesättigte Kohlenwasserstoffrest ein Rest ist, der eine oder zwei Doppelbindungen enthält.

12. Zusammensetzung nach einem der vorangehenden Ansprüche, wobei der pharmakologische Wirkstoff eine niedrige Wasserlöslichkeit aufweist, wie etwa Cyclosporin oder ein Analog davon.

13. Zusammensetzung nach einem der Ansprüche 1 - 12, wobei der pharmakologische Wirkstoff negativ geladen ist, wie etwa ein negativ geladenes Protein, ein negativ geladenes Kohlenhydrat, vorzugsweise Derivate von Heparin mit niedrigem Molekulargewicht, oder DNA.

14. Zusammensetzung nach einem der vorangehenden Ansprüche, welche einen Wassergehalt von höchstens 5 Gew.-%, vorzugsweise höchstens 2 Gew.-% und am meisten bevorzugt höchstens 1 Gew.-% aufweist.

15. Zusammensetzung nach einem der vorangehenden Ansprüche, welche fest, halbfest oder flüssig, vorzugsweise fest oder halbfest ist.

16. Zusammensetzung nach Anspruch 15, welche in Form eines Pulvers oder eines wachsartigen Pulvers, vorzugsweise eines gefriergetrockneten oder sprühgetrockneten Pulvers vorliegt.

17. Zusammensetzung nach Anspruch 15 , welche eine pumpfähige Masse ist, die in eine Kapsel eingefüllt werden kann.

18. Zusammensetzung nach einem der vorangehenden Ansprüche, welche bei Kontakt mit Wasser oder einem anderen wässrigen Medium kolloidale Teilchen bildet, vorzugsweise eine feste, insbesondere wasserfreie Zusammensetzung.

19. Zusammensetzung nach Anspruch 18, wobei der pharmakologische Wirkstoff eine negativ geladene Substanz oder eine Substanz mit einer niedrigen Wasserlösüchkeit ist.

20. Zusammensetzung nach einem der Ansprüche 1 -17, welche bei Kontakt mit Wasser oder einem anderen wässrigen Medium Mizellen, eine Mikroemulsion, eine Emulsion oder eine Dispersion von einer flüssigkristallinen Phase bildet.

21. Zusammensetzung nach einem der Ansprüche 1 - 17, welche bei Kontakt mit Wasser oder einem anderen wässrigen Medium eine Dispersion von einer kubischen, lamellaren oder hexagonalen flüssigkristallinen Phase bildet.

22. Zusammensetzung nach einem der vorangehenden Ansprüche, wobei der Träger auch einen Exzipienten, ausgewählt aus Polymeren, Lipiden, Kohlenhydraten, nichtionischen oberflächenaktiven Verbindungen und Mischungen davon, umfasst.

23. Zusammensetzung nach Anspruch 22, wobei das Kohlenhydrat ein niedermolekulares Kohlenhydrat, vorzugsweise ausgewählt aus Lactose, Sucrose, Maltose und Trehalose ist.

24. Zusammensetzung nach Anspruch 22 oder 23, wobei das Lipid ausgewählt ist aus Phospholipiden, Cholesterin und Glyceriden von mittel- oder langkettigen Fettsäuren.

25. Verwendung eines labilen Esters von Betain und einem lipophilen Alkohol mit wenigstens einer primären Hydroxylgruppe als ein kationischer Exzipient in einem Träger für eine pharmazeutische Zusammensetzung, die einen pharmakologischen Wirkstoff umfasst.

26. Verwendung nach Anspruch 25, wobei der Träger im Wesentlichen nicht-wässrig ist.

27. Verwendung nach Anspruch 25 oder 26, wobei die Zusammensetzung im Wesentlichen nicht-wässrig ist.

28. Verwendung nach einem der Ansprüche 25 - 27, wobei der Alkohol wie in einem der Ansprüche 4 - 11 definiert ist.

29. Verwendung nach einem der Ansprüche 25 - 28, wobei die Zusammensetzung wie in einem der Ansprüche 12 - 24 definiert ist.

## Revendications

1. Composition pharmaceutique comprenant une quantité pharmaceutiquement efficace d'une substance pharmacologiquement active et un support pharmaceutiquement acceptable comprenant un excipient cationique, dans laquelle ledit excipient cationique est un ester labile de bétaïne et d'un alcool lipophile ayant au moins un groupe hydroxyle primaire.

2. Composition selon la revendication 1, dans laquelle ledit support est essentiellement non aqueux.

3. Composition selon la revendication 1 ou 2, ladite composition étant essentiellement non aqueuse.

4. Composition selon l'une quelconque des revendications précédentes, dans laquelle ledit alcool lipophile est un alcool de formule R-CH₂-OH où R est un résidu hydrocarboné aliphatique saturé ou insaturé ayant 7 - 30, de préférence 7 - 22, atomes de carbone.

5. Composition selon la revendication 4, dans laquelle ledit résidu hydrocarboné a au moins un groupe hydroxyle primaire et/ou au moins un groupe hydroxyle secondaire comme substituant(s).

6. Composition selon la revendication 5, dans laquelle lesdits groupes hydroxyle primaires et/ou secondaires sont chacun au plus au nombre de deux.

7. Composition selon l'une quelconque des revendications 4 - 6, dans laquelle ledit résidu hydrocarboné est interrompu par au moins un, de préférence un ou deux, atomes d'oxygène.

8. Composition selon la revendication 7, dans laquelle ledit ou lesdits atomes d'oxygène sont présents dans une liaison ester.

9. Composition selon la revendication 8, dans laquelle ladite liaison ester est présente dans un glycéride.

10. Composition selon la revendication 9, dans laquelle ledit glycéride est un 1-monoglycéride ou un 2-monoglycéride.

11. Composition selon l'une quelconque des revendications 4 - 10, dans laquelle ledit résidu hydrocarboné insaturé est un résidu contenant une ou deux doubles liaisons.

12. Composition selon l'une quelconque des revendications précédentes, dans laquelle ladite substance pharmacologiquement active a une faible solubilité dans l'eau, comme une cyclosporine ou un de ses analogues.

13. Composition selon l'une quelconque des revendications 1 - 12, dans laquelle ladite substance pharmacologiquement active est chargée négativement, comme une protéine chargée négativement, un hydrate de carbone chargé négativement, de préférence un dérivé de faible masse molaire de l'héparine, ou un ADN.

14. Composition selon l'une quelconque des revendications précédentes, qui a une teneur en eau d'au plus 5 % en masse, de préférence d'au plus 2 % en masse et au mieux d'au plus 1 % en masse.

15. Composition selon l'une quelconque des revendications précédentes, qui est solide, semi-solide ou liquide, de préférence solide ou semi-solide.

16. Composition selon la revendication 15, qui est sous forme d'une poudre ou d'une poudre cireuse, de préférence d'une poudre lyophilisée ou séchée par atomisation.

17. Composition selon la revendication 15, qui est une masse pompable qui peut être introduite dans une capsule.

18. Composition selon l'une quelconque des revendications précédentes, qui, au contact avec de l'eau ou un autre milieu aqueux, forme des particules colloïdales, de préférence une composition solide, en particulier anhydre.

19. Composition selon la revendication 18, dans laquelle ladite substance pharmacologiquement active est une substance chargée négativement ou une substance ayant une faible solubilité dans l'eau.

20. Composition selon l'une quelconque des revendications 1 - 17, qui, au contact avec de l'eau ou un autre milieu aqueux, forme des micelles, une microémulsion, une émulsion ou une dispersion d'une phase de cristaux liquides.

21. Composition selon l'une quelconque des revendications 1 - 17 qui, au contact avec de l'eau ou un autre milieu aqueux, forme une dispersion d'une phase de cristaux liquides cubiques, lamellaires ou hexagonaux.

22. Composition selon l'une quelconque des revendications précédentes, dans laquelle ledit support comprend aussi un excipient choisi parmi des polymères, des lipides, des hydrates de carbone, des composés tensioactifs non ioniques et leurs mélanges.

23. Composition selon la revendication 22, dans laquelle ledit hydrate de carbone est un hydrate de carbone de faible masse molaire, choisi de préférence parmi le lactose, le sucrose, le maltose et le tréhalose.

24. Composition selon la revendication 22 ou 23, dans laquelle ledit lipide est choisi parmi des phospholipides, le cholestérol et des glycérides d'acides gras à chaîne moyenne ou longue.

25. Utilisation d'un ester labile de bétaïne et d'un alcool lipophile ayant au moins un groupe hydroxyle primaire comme excipient cationique dans un support pour une composition pharmaceutique comprenant une substance pharmacologiquement active.

26. Utilisation selon la revendication 25, dans laquelle ledit support est essentiellement non aqueux.

27. Utilisation selon la revendication 25 ou 26, dans laquelle ladite composition est essentiellement non aqueuse.

28. Utilisation selon l'une quelconque des revendications 25 - 27, dans laquelle ledit alcool est tel que défini dans l'une quelconque des revendications 4 - 11.

29. Utilisation selon l'une quelconque des revendications 25 - 28, dans laquelle ladite composition est telle que définie dans l'une quelconque des revendications 12 - 24.
